# EUROPEAN PATENT APPLICATION

(11) **EP 0 990 637 A1**
(43) Date of publication of application: **05.04.2000**
(21) Application number: 98917708.4
(22) Date of filing: 28.04.1998
(51) Int. Cl.: C07C 59/84, C07C 51/06, C07C 51/377, C07D 209/34, C07B 61/00

(54) **PROCESS FOR PRODUCING KETOPROFEN AND 5-BENZOYL-3-METHYL-2-INDOLINONE**

(30) Priority: 28.04.1997 JP 11123697; 04.08.1997 JP 20927897
(71) Applicant: Medical Information Services, Inc., Tokyo104-0041 (JP)
(72) Inventor: KONDO, Hisao, Kanagawa 248-0036 (JP); HIGASHIKAWA, Tetsuro, Tokyo 153-0051 (JP); KUBO, Akinari, Tokyo 152-0021 (JP); SAITO, Naoki, Kanagawa 215-0011 (JP)
(74) Representative: Reinhard - Skuhra - Weise & Partner
(86) International application number: JP9801939
(87) International publication number: WO9849127

(57) **Abstract**

The present invention relates to a process for producing ketoprofen, comprising the steps of: benzoylating 3-methyl-2-indolinone with benzoyl halide to yield 5-benzoyl-3-methyl-2-indolinone; cleaving an amido linkage that forms the 5-menbered ring moiety of 5-benzoyl-3-methyl-2-indolinone, with a basic compound; and substituting an amino group attached to an aromatic ring of the resulting compound by a hydrogen atom to yield the ketoprofen. By employing the process, it is possible to produce ketoprofen by ordinary steps without the need for use of any extremely poisonous chemical such as prussic acid compounds or any hard-to-handle chemical such as liquid ammonium, by more shortened steps as a whole, with an excellent yield.

## Description

### [TECHNICAL FIELD]

The present invention relates to a process for producing ketoprofen.

Ketoprofen has a chemical name 2-(3-benzoylphenyl)propionic acid and has been used in the pharmaceutical field as an anti-inflammatory agent.

For the production of ketoprofen, 5-benzoyl-3-methyl-2-indolinone is a key substance.

### [Background Art]

For the production of ketoprofen, there has been proposed various processes. Among these, typical processes are as follows:
(1) a process in which 3-methylbenzophenone is brominated to yield 3-bromomethylbenzophenone; the 3-bromomethylbenzophenone is then reacted with potassium cyanide to yield 3-cyanomethylbenzophenone; and the 3-cyanomethylbenzophenone is methylated with methyl iodide in the presence of a base and then subjected to alkaline hydrolysis to yield ketoprofen (see JP-A-51-115452);
(2) a process in which 3-chlorobenzoic acid is reacted with propionitrile in the presence of a strong base to yield (3-carboxyphenyl)propionitrile; the (3-carboxyphenyl)propionitrile is reacted with thionyl chloride to yield (3-chlorocarbonylphenyl)propionitrile; the (3-chlorocarbonylphenyl)propionitrile is subjected to the Friedel-Crafts reaction with benzene in the presence of aluminum chloride to yield 3-(1-cyanoethyl)benzophenone; and the 3-(1-cyanoethyl)benzophenone is subjected to alkaline hydrolysis to yield ketoprofen (see J. Org. Chem., 31, 602 (1966), and JP-B-52-8301); and
(3) a process in which aniline is reacted with 2-chloropropionyl chloride to yield acid anilide; the acid anilide is cyclized using aluminum chloride as a catalyst to yield 3-methyl-2-indolinone; the 3-methyl-2-indolinone is benzylated with benzyl chloride to yield 5-benzyl-3-methyl-2-indolinone; the 5-benzyl-3-methyl-2-indolinone is subjected to alkaline hydrolysis to yield 2-(3-benzyl-6-aminophenyl)propionic acid; the amino group of the 2-(3-benzyl-6-aminophenyl)propionic acid is substituted by a hydrogen atom to yield 2-(3-benzylphenyl)propionic acid; and the 2-(3-benzylphenyl)propionic acid is oxidized to yield ketoprofen (see JP-A-64-66144).

However, the process (1) is not suitable for industrial purpose, because the process utilizes poisonous potassium cyanide for the synthesis of 3-cyanomethyl-benzophenone.

The process (2) is also disadvantageous, because hard-to-handle liquid ammonia is utilized and the yield in the step for the synthesis of (3-carboxyphenyl)propionitrile is as poor as 50% or less.

The process (3) requires a step for oxidizing the benzylated methylene group. Moreover, the yield in this step is as poor as about 47%, the process is very long as a whole, and the total yield of the process is extremely low. These defects are fatal for an industrial purpose. Thus, the production of ketoprofen is accompanied with some difficulties, resulting in expensive production cost.

Therefore, the object of the present invention is to provide a process for producing ketoprofen which can overcome the above-mentioned problems. That is, this process comprises ordinary steps but does not need use of extremely poisonous chemicals (e.g., prussic acid compounds) or hard-to-handle chemicals (e.g., liquid ammonium), and it has more shortened steps as a whole and can produce ketoprofen in a good yield.

Among the prior processes as mentioned above, the process of Japanese Patent Application Laid-open No. 64-66144 is a process in which 3-methyl-2-indolinone is benzylated, followed by hydrolysis, deamination and oxidation, thereby yielding ketoprofen. This process is superior to other prior processes in that the use of prussic acid-based compounds or hard-to-handle compounds (e.g., liquid ammonium) is not needed. However, this process requires a step for oxidizing the methylene group. As a result, the process becomes long as a whole, and the yield in the oxidizing step is as poor as 50% or less, resulting in lowering of the total yield in this process. Due to these reasons, it has been very difficult to put this process into practical use as an industrial process.

We have intensively studied for the purpose of overcoming the above-mentioned problems. As a result, we have now found that it is possible to produce ketoprofen without the oxidation step that renders it hard to raise a yield of ketoprofen, by benzoylating 3-methyl-2-indolinone rather than benzylating the same, cleaving the amido linkage on the 5-menbered ring, and then deaminating the amido linkage-derived amino group that is attached to an aromatic ring of the resulting compound. This finding lead us to accomplishment of the invention.

### [DISCLOSURE OF THE INVENTION]

The process for producing ketoprofen according to the present invention overcoming the above-mentioned problems, comprises:
benzoylating 3-methyl-2-indolinone with benzolyl halide to yield 5-benzoyl-3-methyl-2-indolinone;
cleaving an amido linkage, which forms the 5-menbered ring moiety of 5-benzoyl-3-methyl-2-indolinone, with a basic compound; and
substituting an amino group attached to the aromatic ring of the resulting compound by a hydrogen atom to yield ketoprofen.

An example of the process for producing ketoprofen according to the present invention will be described below.

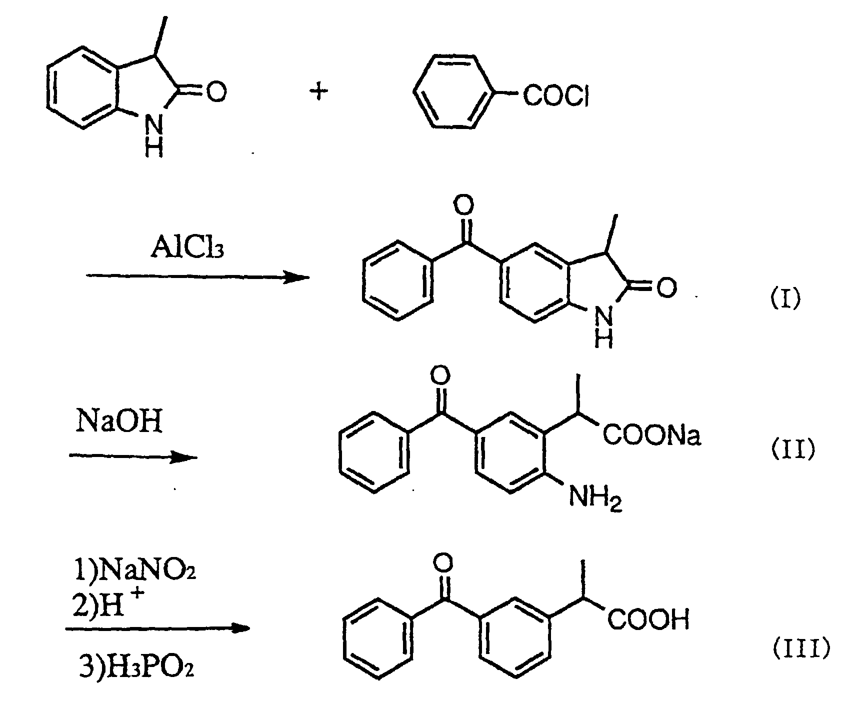

In the above reaction scheme, the starting material 3-methyl-2-indolinone is known to be synthesized by several processes. Example of typical processes are: (1) a process in which 2-(2-nitrophenyl)propionic acid is reduced (Ann., 227, 274 (1885)); (2) a process in which 2-propionylphenylhydrazine is treated with calcium halide under heating (Org. Syn., Col1. Vol. IV 657); and (3) a process in which 2-chloropropionyl anilide is treated with aluminum chloride (JP-A-64-66144; Tetrahedon, 24, 6093 (1968)).

The synthesis of 5-benzoyl-3-methyl-2-indolinone by reaction of 3-methyl-2-indolinone with benzoyl halide is performed in the presence of an appropriate Friedel-Crafts catalyst such as aluminum chloride. (This reaction corresponds to step (I) in the above-described reaction formula.)

This reaction itself is already known, and JP-A-57-26585 proposes a process which comprises heating a slurry comprising 5 moles of aluminum chloride and 3 moles of benzoyl chloride per mole of 3-methyl-2-indolinone at 180°C-185°C, adding 3-methyl-2-indolinone to the slurry, and benzoylating 3-methyl-2-indolinone.

However, the process described indeed in this Japanese application is only a process utilizing 2-indolinone as a starting material, as illustrated in its working example, and in its Production Example 2 there is a description that benzoylation of 3-methyl-2-indolinone could be performed in the same manner as in this process. In this working example, large amounts of aluminum chloride (5 moles per mole of 3-methyl-2-indolinone) and benzoyl chloride (3 moles per mole of 3-methyl-2-indolinone) are used, and the reaction is performed at a temperature of 180-185°C. When we attempted to benzolylate 3-methyl-2-indolinone according to the procedures of the working example, a substance insoluble in water or organic solvents was generated after the reaction treatment, the yield was poor, and there were many problems regarding production (see Reference Example 1 below). 5-Benzoyl-3-methyl-2-indolinone is an important intermediate for the production of ketoprofen and is also expected to take a role as an intermediate for production of various fine chemicals in view of its structure. However, in fact, industrial process for the production of 5-benzoyl-3-methyl-2-indolinone has not yet been established.

We have intensively studied for the purpose of overcoming defects of the above-mentioned reactions. As a result, we have now found that good results can be obtained by use of 2 moles or more of aluminum chloride and 1.5 moles or more of benzoyl chloride per mole of 3-methyl-2-indolinone and of a reaction temperature of 100°C or lower.

By maintaining such reaction conditions, any by-product having a high molecular weight (e.g., solid materials insoluble in organic solvents or water, or compounds evaluated to be highly polar than the target product 5-benzoyl-3-methyl-2-indolinone by thin layer chromatography), is formed in a very small amount or is almost not formed; the yield of 5-benzoyl-3-methyl-2-indolinone is as high as 70-80%; and the product can be isolated easily. Thus, this process is suitable for industrial practice. It is preferable to employ a reaction temperature of 80°C or lower, because such a temperature enables to reduce formation of polar products to a lower level undetectable by thin layer chromatography.

The above-mentioned technique according to the invention is a process where solid 3-methyl-2-indolinone, which is previously isolated and purified, is used as a raw material. For example, this process may comprising heating 2-halopropionyl anilide represented by formula (IV) below in the presence of aluminum halide; adding benzyl halide directly to the resulting reaction mixture without separation of the produced 3-methyl-2-indolinone; and finally obtaining ketoprofen through production of 5-benzoyl-3-methyl-2-indolinone. This process is advantageous in that ketoprofen can be produced from cheaper materials by more shortened steps with no need for separating 3-methyl-2-indolinone. Hereinafter, the process for the production of ketoprofen from the raw material 2-halopropionyl anilide through an intermediate 5-benzoyl-3-methyl-2-indolinone will be described in detail.

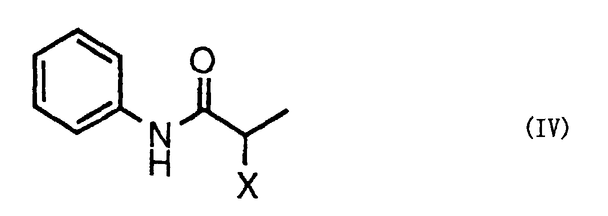

The raw material 2-chloropropionyl anilide (Formula (IV)) may be synthesized readily by condensing 2 moles of commercially available aniline with, for example, 1 mole of 2-chloropropionyl chloride in the presence of an appropriate organic diluent, as shown in reaction formula (V). Of the 2 moles of aniline, 1 mole is used for capturing a generated hydrogen halide, and the hydrohalide salt of aniline is separated, followed by its neutralization with an alkali, to recover it as aniline.

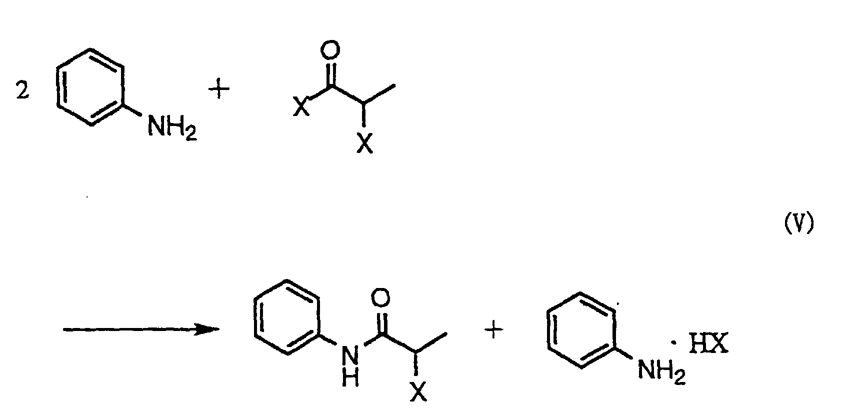

The 2-chloropropionyl anilide obtained above is used as a raw material for the production of 5-benzoyl-3-methyl-2-indolinone, as shown in the subsequent reaction formula (VI). 5-benzoyl-3-methyl-2-indolinone is then treated in the same way as in synthesis examples (1)-(3) of ketoprofen from 5-benzoyl-3-methyl-2-indolinone as described below, thereby finally yielding ketoprofen. In formulae, "X" represents a halogen atom and "Ph" represents a phenyl group.

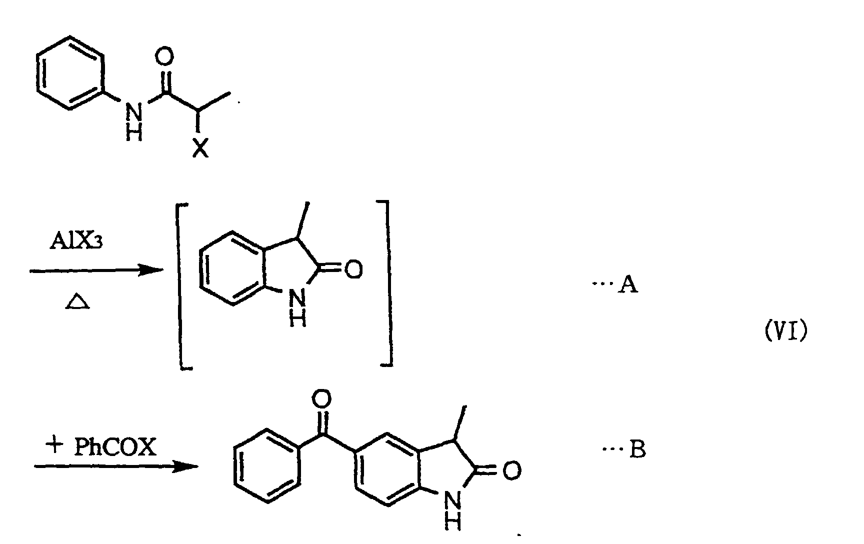

In the two-step reaction shown in reaction formula (VI), step A is the synthesis of 3-methyl-2-indolinone through cyclization and dehydrogen halide of 2-chloropropionyl anilide, the synthesis being usually achieved by heating 2-chloropropionyl chloride in the presence of aluminum halide. The aluminum halide is preferably aluminum chloride (anhydrous) in view of reactivity and economy.

In step A, the molar ratio of aluminum halide to 2-chloropropionyl anilide is 1:1 or more, and the reaction temperature is 120°C or higher. Preferably, the molar ratio is 1:1.2 or more (i.e., excess 2-chloropropionyl anilide) and the reaction temperature is from 130°C to 185°C. Solvent may not necessarily be used, but when used, a solvent has to have a high boiling point and be usable for the Friedel-Crafts reaction. Preferably, the solvent is dichlorobenzene.

In the reaction formula (VI), step (B) is the synthesis of 5-benzoyl-3-methyl-2-indolinone by reaction of 3-methyl-2-indolinone with benzoyl halide. This reaction is performed under the same reaction conditions as those for preparation of 5-benzoyl-3-methyl-2-indolinone using the above-mentioned isolated and purified 3-methyl-2-indolinone as a starting material, namely, in the presence of an appropriate Friedel-Crafts catalyst, aluminum halide. The aluminum halide used is preferably aluminum chloride (anhydrous) in view of reactivity and economy.

The reaction of step B may usually be achieved by adding dropwise benzoyl halide to a mixture of 3-methyl-2-indolinone and aluminum halide and then heating. The benzoyl halide is required to have a sufficient reactivity, and is preferably benzoyl chloride in view of economy.

The amount of benzoyl chloride added may be equimolar with or greater than 2-chloropropionyl anilide, and preferably 1.2 times or more the molar amount of the 2-chloropropionyl anilide. The amount of aluminum halide catalyst may be 1.2 times or more, preferably 1.5 times or more, the molar amount of 2-chloropropionyl anilide. The reaction may be performed without solvent, but in this case the reaction mixture becomes so viscous that stirring becomes hard. Hence, it is preferable to use a small amount of a solvent. Solvents suitable for the Friedel-Crafts reaction, such as chlorine-containing hydrocarbons (e.g., 1,2-dichloroethane) and carbon disulfide, are preferably chosen.

In the process as mentioned above, it becomes possible to employing a same solvent in both steps A and B of the reaction formula (VI), resulting in saving the amount of aluminum halide used and in eliminating a step for isolating 3-methyl-2-indolinone after the completion of step A, thereby achieving more shortened steps. The reaction temperature for step B is preferably set between a temperature at which hydrogen halide is generated and a boiling point (i.e., a reflux temperature) of a solvent, and preferably 100°C or lower. The temperature of 80°C or lower is more preferable, because the amount of by-products can be reduced.

In the process for producing 5-benzoyl-3-methyl-2-indolinone shown in reaction formula (VI), if it is required to perform benzoylation without separating 3-methyl-2-indolinone, the step A may be firstly performed and then the step B may be performed using the system obtained after the completion of step A. Preferably, the amount of anhydrous aluminum chloride added to the reaction system in step A is chosen taking into account the suitable amount of anhydrous aluminum chloride used in step (B). That is, the step A is performed, the resulting reaction mixture is cooled, and then benzoyl chloride is added dropwise thereto. Since heat is generated during the addition of benzoyl chloride, this addition should be carried out at a temperature not exceeding 80°C. After the addition was completed, it is preferable that the benzoylation is carried out while maintaining optimum conditions for step B.

After the reaction, the reaction system is cooled, and dissolved in an inert diluting solvent. Subsequently, ice blocks, ice cold water or cooled water may be added dropwise to the resulting reaction mixture while cooling on ice, or alternatively, the reaction mixtuer is added dropwise to ice blocks, ice cold water or cooled water while cooling on ice, thereby inactivating the active aluminum chloride. As such diluting solvents, preferred are solvents having some polarity and capable of dissolving the reaction mixture, such as ethyl acetate and 1,2-dichloroethane.

During the inactivation of aluminum chloride mentioned above, solids of an aluminum chloride-containing salt is precipitated. In this case, water is added to the reaction system until the solids are completely dissolved, and then the aqueous phase is separated. The organic phase is washed with water, subsequently with an aqueous alkali solution, and dried over anhydrous magnesium sulfate and potassium carbonate, and then the solvent is evaporated. To the residue is added a lower alcohol, preferably ethanol or methanol. This solution is seeded for crystallization with crystals of purified 5-benzoyl-3-methyl-2-indolinone, and allowed to stand overnight while cooling on ice to cause the crystallization of 5-benzoyl-3-methyl-2-indolinone. After filtered, the obtained crystalline material is further purified by re-crystallization to give 5-benzoyl-3-methyl-2-indolinone in a pure form.

So-obtained 5-benzoyl-3-methyl-2-indolinone is treated with a basic compound to cleave the amido linkage in its 5-menbered ring moiety.

The basic compound used may be any one as long as it is capable of cleaving the amido linkage. From the viewpoint of solubility in water and economy, sodium hydroxide or potassium hydroxide is preferred.

When sodium hydroxide or potassium hydroxide is used, an alkali salt of 2-(5-benzoyl-2-aminophenyl)propionic acid may be generated. (This reaction corresponds to step (II) of the aforementioned reaction formula.)

In the hydrolysis with an alkali, the amount of sodium hydroxide or potassium hydroxide used is preferably 1 mole or more, more preferably from 1.2 moles to 2 moles, based on 1 mole of 5-benzoyl-3-methyl-2-indolinone. In addition, in order to increase the rate of the hydrolysis, a small amount of a water-soluble aliphatic alcohol, preferably ethanol, may be incorporated.

To obtain ketoprofen, it is necessary to substitute the amino group attached to the phenyl group by a hydrogen atom. According to the conventional methods, such substitution is usually performed by adding an amino compound to an acidic solution, further adding dropwise a nitrite to cause diazotization, and then reducing the diazotized compound with a reducing agent, thereby replacing the amino group by a hydrogen atom.

However, the 2-(5-benzoyl-2-aminophenyl)propionic acid prepared by neutralizing the above-described alkali salt of 2-(5-benzoyl-2-aminophenyl)propionic acid with an acid is unstable and, after generated, this compound re-cyclizes readily to revert into the starting 5-benzoyl-3-methyl-2-indolinone. Hence, the substitution of the amino group by a hydrogen atom could not carried out by the above-mentioned conventional method; that is, this substitution by the usual method was difficult.

However, we have now found that, in the process in which the amide linkage that forms the 5-menbered ring moiety of 5-benzoyl-3-methyl-2-indolinone is cleaved with a basic compound and the aromatic amino group of the resulting compound is substituted by a hydrogen atom, the cleavage of the amido linkage and the substitution of the generated amino group by a hydrogen atom can be achieved readily and surely by cleaving amido linkage with an alkali metal hydroxide to cause hydrolysis, adding a nitrite thereto, adding dropwise the resulting solution to an acidic solution to cause diazotization, and reducing the diazotized compound with a reducing agent.

Using the above-mentioned procedures, the end product of the process, ketoprofen, can be produced efficiently by producing 2-(5-benzoyl-2-aminophenyl)propionic acid from an alkali salt thereof and then diazotizing the 2-(5-benzoyl-2-aminophenyl) propionic acid immediately.

The nitrite used in the diazotization is preferably sodium nitrite or potassium nitrite from the viewpoint of solubility in water and economy. The amount of the nitrite used is preferably from 1.05 to 1.2 times the molar amount of the alkali salt of 2-(5-benzoyl-2-aminophenyl)propionic acid. If the amount is less than 1.05 times, the diazotization may not proceed satisfactory, whereas if the amount is more than 1.2 times, the improvement in the yield of the desired compound is not observed and any economical benefit cannot be given.

The above-mentioned acidic solution is preferably a mineral acid, such as sulfuric acid or hydrochloric acid. This is because such an acid can provide a sufficient acidity and make the purification of the desired product ready in the subsequent step. Subsequently, a solution containing the alkali salt of 2-(5-benzoyl-2-aminophenyl)propionic acid and the nitrite is added dropwise to the acidic solution to cause the diazotization. The temperature employed in this step may be within the range where the generated diazonium salt can be present stably, and usually from -15°C to 15°C. If the temperature is lower than -15°C, the reaction is likely to proceed slower, whereas if the temperature is higher than 15°C, the generated diazonium salt is likely to be decomposed thereby causing a decrease of the yield.

As mentioned above, there are known various methods for reducing the diazonium salt given by the diazotization. Among them, hypophosphorous acid or a salt thereof is preferably employed. Their amounts used may be 2.5 moles or more per mole of an alkali salt of 2-(5-benzoyl-2-aminophenyl) propionic acid, which amounts enable to proceed the reduction reaction sufficiently.

After the addition of hypophosphorous acid or a salt thereof as mentioned above, for the purpose of facilitating the rapid proceeding of the reaction, an appropriate catalyst, such as copper powder, cuprous oxide and a cuprous salt, may be incorporated. The amount of the catalyst is preferably within the range from of 1 to 3 wt% inclusive based on the amount of the 2-(5-benzoyl-2-aminophenyl) propionic acid.

After the addition of hypophosphorous acid or its salt and, optionally, a catalyst, the reaction system is warmed to room temperature to complete the reduction reaction. (This step corresponds to step (III) in the aforementioned reaction formula.)

### [BEST MODE FOR CARRYING OUT THE INVENTION]

Hereinbelow, the present invention will be described in more detail with reference to the following Examples. However, it should be understood that the invention is not limited to those examples.

### Reference Example 1 (JP-B-57-26585; Supplemental test for Production Example-2)

A mixture of aluminum chloride (66 g, 0.5 mol) and benzoyl chloride (42.5 g, 0.3 mol) was heated to 150°C, and 3-methyl-2-indolinone (14.7 g, 0.1 mol) was added thereto in limited amounts while keeping the temperature within the range of 180-185°C to allow to react these compounds. After the addition, the reaction solution was stirred for 5 minutes while keeping the temperature at 170°C. The reaction solution was cooled to room temperature, added to ice cold water to inactivate the catalyst ammonium chloride, and then extracted with ethyl acetate (100 ml). As a result, a solid material insoluble in both ethyl acetate and water was yielded in a large amount. This solid material is assumed to be by-products having a high molecular weight. The treated solution was filtered to remove the solid material, and the filtrate was fractionated to separate the ethyl acetate phase.

The organic phase was washed with water and subsequently with an aqueous sodium hydroxide solution to decompose excess benzoyl chloride. The resulting solution was further washed with water, then dried over anhydrous magnesium sulfate. After removal of the magnesium sulfate by filtration, ethyl acetate was evaporated. To the residue was added methanol to crystallize the target product. The crystals were filtered, washed with methanol and then dried (yield: 8.00 g). Separately, methanol was evaporated from the filtrate, and the residue was added with a small volume of methanol again to dissolve the residue. The resulting solution was cooled on ice to separate out crystals. The crystals were collected by filtration, washed with cold methanol, and dried (yield: 3.67 g). The total yield of the product was 11.67 g (46.5%).

### Synthesis Example for 5-benzoyl-3-methyl-2-indolinone (Example of the use of isolated and purified 3-methyl-2-indolinone - (1))

Benzoyl chloride (0.7 ml, 0.06 mol) was added dropwise to a suspension of aluminum chloride (0.988 g, 0.075 mol) in 1,2-dichloroethane (5 ml) over 5 minutes while stirring and cooling on ice, and then stirred for additional 1 hour. A solution of 3-methyl-2-indolinone (0.441 g, 0.03 mol) in 1,2-dichloroethane (5 ml) was added dropwise to the resulting solution over 10 minutes. The resulting reaction solution was refluxed with heating in an outer bath at 110°C for 24 hours.

After cooling, the reaction solution was poured into ice cold water (100 g), and extracted twice with methylene chloride (50 ml). The organic phases were combined together, washed with water, and dried. After evaporation of the solvent, the residue (1.0 g) was crystallized from methanol to yield crystals (0.282 g). The mother liquor (0.6451 g) was subjected to a column chromatography on silica gel (35 g). From the fraction eluted with benzene-ethyl acetate (5:1), the desired substance (0.116 g) was obtained.

Total: 0.398 g (yield: 52.8%). mp: 181-182.5°C.

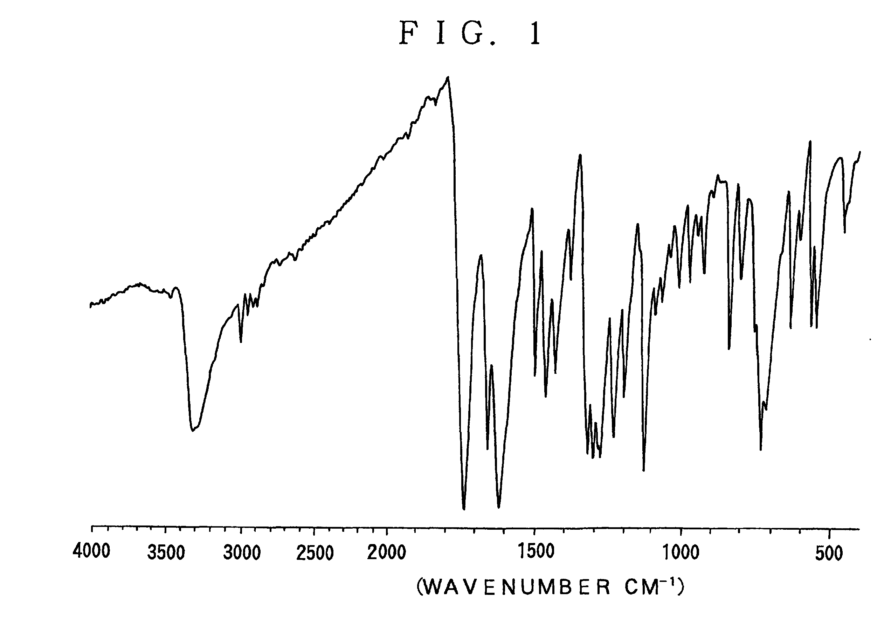

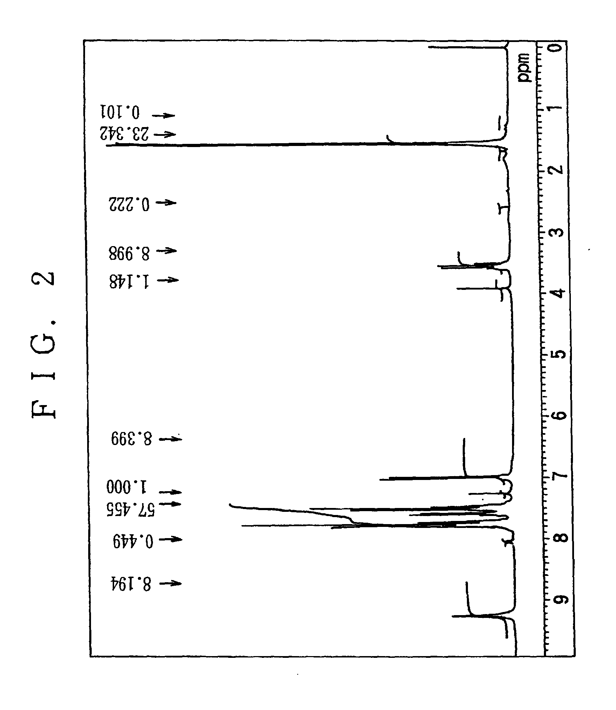

From IR (Fig. 1) and ¹H-NMR spectra (Fig. 2, solvent: CDCl₃), the substance obtained was identified to be 5-benzoyl-3-methyl-2-indolinone.

### (Example of the use of isolated and purified 3-methyl-2-indolinone - (2))

Anhydrous aluminum chloride (10.01 g, 0.075 mol) was added to 3-methyl-2-indolinone (5.30 g, 0.036 mol), and then anhydrous benzoyl chloride (7.65 g, 0.054 mol) was slowly added thereto at room temperature. After the addition, the resulting reaction solution was heated to 95°C over 30 minutes. During this heating, the crystalline substances were dissolve at about 70-80°C and the reaction was started with generation of hydrogen chloride. The reaction solution was stirred for 2 hours while keeping the temperature at 95°C. After the reaction was completed, the reaction solution was cooled to 80°C and dissolved in ethyl acetate (70 ml). The reaction solution was cooled to room temperature, and water was added thereto slowly while cooling on ice to inactivate the aluminum chloride, whereby a solid substance that was assumed to be a hydrate of aluminum chloride was precipitated. During this treatment, since the reaction proceeded violently with generation of heat, the procedure was performed very carefully. Water was added to the reaction solution to dissolve solids. Following fractionateion, the organic phase was washed twice with water (50 ml), and the ethyl acetate was removed from the solution using an evaporator until the solution was condensed to about 20 ml. The condensed solution was added with n-hexane (100 ml) and allowed to stand, thereby causing precipitation of crystals. After filteration, the filter cake was washed with a mixed solution (20 ml) of ethyl acetate:n-hexane (1:1), further with n-hexane (20 ml), and dried, thereby yielding 5-benzoyl-3-methyl-2-indolinone (yield: 7.54 g, 80.1%).

### (Example of the use of isolated and purified 3-methyl-2-indolinone - (3))

Benzoyl chloride (28.1 g, 0.2 mol) was added dropwise slowly to a mixture of 3-methyl-2-indolinone (14.7 g, 0.1 mol) and anhydrous aluminum chloride (40.0 g, 0.3 mol) at room temperature. During this addition, generation of heat was observed. After the dropwise addition was completed, 1,2-dichloroethane (20 ml) was added to the reaction solution, and heated to 75-80°C. After 5 hours, ethyl acetate (50 ml) was added to the reaction solution while cooling with ice cold water (during this addition, heat was generated), to prepare a homogeneous solution. Ice blocks were was added to the solution in limited amounts to inactivate the aluminum chloride. The lastly precipitated solid substance that was assumed to be a hydrate of aluminum chloride was dissolved completely by the addition of water. During this procedure, 5-benzoyl-3-methyl-2-indolinone was precipitated as crystals.

The reaction system was added with hexane (50 ml), stirred, and filtered. The obtained crystalline substance was fully washed with water, further with hexane, and then dried. When the filtered crystalline product was subjected to TLC analysis, only a single spot for 5-benzoyl-3-methyl-2-indolinone was detected.

Following drying, 22.27 g of 5-benzoyl-3-methyl-2-indolinone was obtained. This product was dissolved in ethyl acetate, a small amount of celite was added, and the mixture was filtered. The filtrate was condensed to 50 ml, and hexane (100 ml) was added thereto to crystallize 5-benzoyl-3-methyl-2-indolinone. The crystals were filtered, washed with hexane, and then dried. Yield: 20.0 g (79.7%). The structure was identified by the NMR spectrum.

### (Examples of the use of isolated and purified 3-methyl-2-indolinone - (4)-(6))

Substantially the same procedures as in the section of "Synthesis for 5-benzoyl-3-methyl-2-indolinone [Example of the use of isolated and purified 3-methyl-2-indolinone - (3)]" was repeated, except that the amounts of the individual raw materials charged, the amount of the solvent, the reaction temperature and the reaction time as indicated in Table 1 below were employed. The amounts and the yields of the individual products are also shown in Table 1.

In Table 1, "MI" is 3-methyl-2-indolinone, "AlCl₃" is anhydrous aluminum chloride, "BzCl" is benzoyl chloride, "BMI" is 5-benzoyl-3-methyl-2-indolinone, "DCE" is 1,2-dichloroethane, and "DCB" is o-dichlorobenzene.

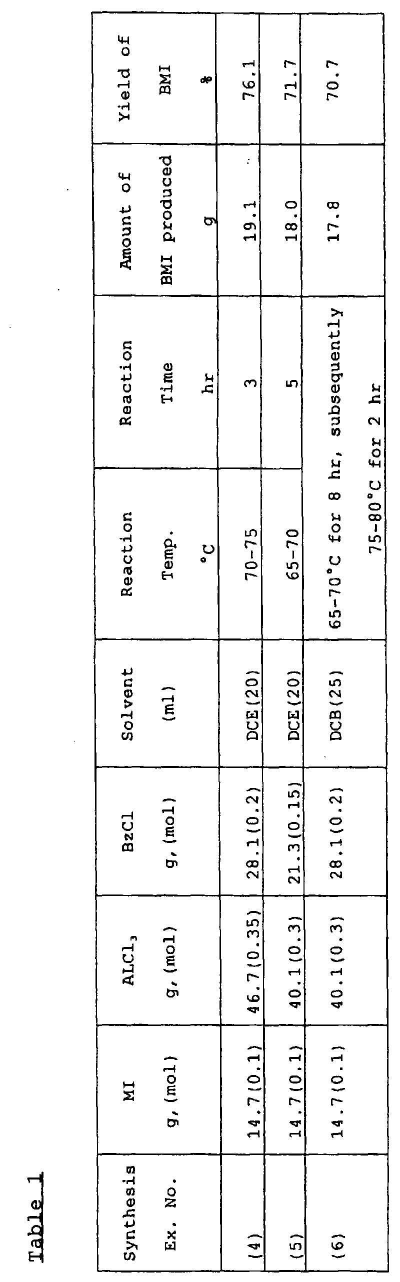

### (Example of the use of unpurified 3-methyl-2-indolinone synthesized from 2-halopropionyl anilide) [See the section of "Synthesis of 2-chloropropionyl anilide: reaction formula (V)]

Aniline (200.2 g, 2.12 mol) was dissolved in ethyl acetate (500 ml), and 2-chloropropionyl chloride (127.5 g, 1.0 mol) was added dropwise thereto over 2-hours while stirring and cooling on ice and then stirred at 30°C for another 1 hour. An aqueous solution (100 ml) of concentrated hydrochloric acid (20 ml) dissolved in water was added to the reaction solution, and stirred for the purpose of dissolving the precipitated aniline hydrochloride. The mixture was fractionated, and washed twice with water (150 ml). Each aqueous phase was re-extracted with ethyl acetate (100 ml), and the extract was washed with water to give an organic phase, which was dried over anhydrous magnesium sulfate, filtered, and concentrated by evaporation of the solvent. The residue was added with hexane (400 ml) to cause the precipitation of crystals. After filtration, the filter cake was washed with hexane and then dried. The filtrate was concentrated in order to re-crystallize the target product from hexane, and the resulting crystals were treated in the same manner as mentioned above to yield a white product (i.e., 2-chloropropionyl anilide).

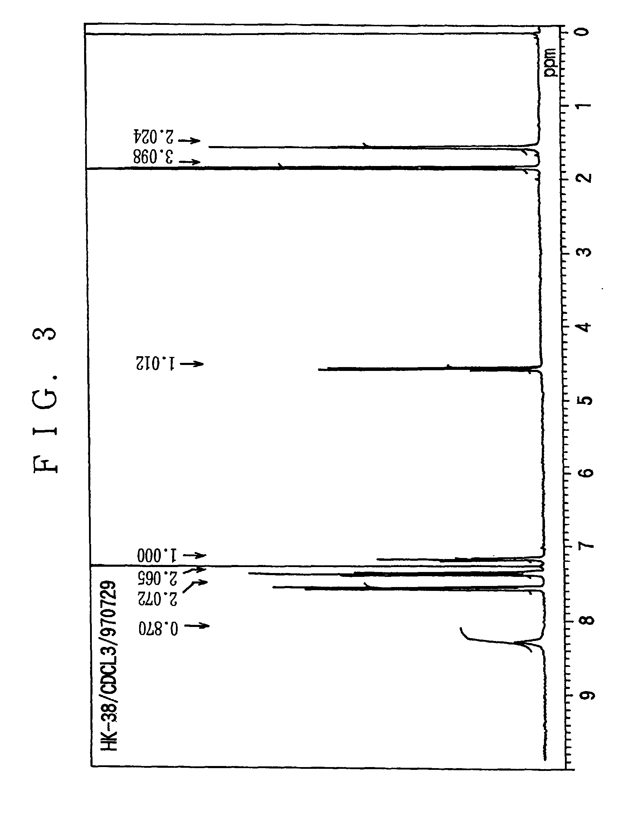

Yield: 158.4 g (85.2%). The structure was identified by the ¹H-NMR spectrum (solvent: CDCl₃) (Fig. 3).

### [Synthesis of 5-benzoyl-3-methyl-2-indolinone from 2-chloropropionyl anilide - (1); see reaction formula (VI)]

Under the atmosphere of an inert gas (argon gas), the above obtained 2-chloropropionyl anilide (36.7 g, 0.2 mol) and aluminum chloride (anhydrous) (special grade; Wako Pure Chemical Industries, Ltd.; a 500-g product; 53.4 g, 0.4 mol) were stirred with heating initially at 130-135°C for 4 hours, subsequently at 150-170°C for 2 hours, and finally at 180°C until the generation of hydrogen chloride gas ceased. In the heating step, white smoke was produced at around 100°C when the compounds began to dissolve, and the temperature was elevated. Moreover, during the reaction, white crystals were deposited on the inner wall of the reactor.

Subsequently, the reaction solution was cooled, and a solution of benzoyl chloride (43.5 g, 0.3 mol) in 1,2-dichloroethane (20 ml) was added dropwise thereto. Because heat was generated during the addition, care was taken so that the temperature does not exceed 80°C. After the addition was completed, the reaction solution was heated to 80-85°C while stirring. Then, the reaction was continued at 90°C until the generation of hydrogen chloride gas ceased. The reaction solution was cooled and dissolved in ethyl acetate (150 ml), and the mixture was cooled on ice and added with a small volume of ice cold water while stirring, thereby inactivating the aluminum chloride. During this step, violent exothermic reaction was observed. Although solids were precipitated, they were dissolved in water completely. After fractionation, the organic phase was washed twice with water (100 ml each), and subsequently with an alkali solution containing sodium hydroxide (8 g, 0.2 mol).

Each aqueous phase obtained was extracted again with ethyl acetate (100 ml), and the organic phases was combined together and dried over anhydrous magnesium sulfate (10 g) and potassium carbonate (10 g). Following filteration, the drying agents were washed with ethyl acetate, then the ethyl acetate was distilled away from the organic phase using an evaporator. The residue was added with ethanol (20 ml), and the mixture was seeded for crystallization with crystals of standard 5-benzoyl-3-methyl-2-indolinone (about 5 mg), and allowed to stand for about 24 hours while cooling on ice. The crystals generated were filtered, washed with cold methanol and dried.

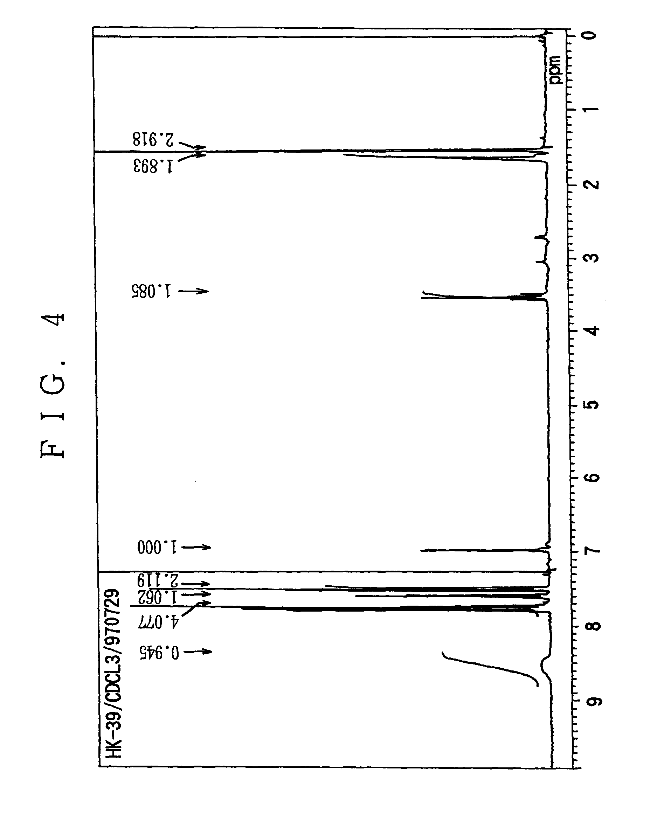

The yield of the crude 5-benzoyl-3-methyl-2-indolinone: 19.63 g (38.6%). Re-crystallization from methanol. Yield: 15.28 g (30.1%). The structure was identified by the ¹H-NMR spectrum (solvent: CDCl₃)(Fig. 4).

### [Synthesis of 5-benzoyl-3-methyl-2-indolinone from 2-chloropropionyl anilide - (2)]

In the same manner as described in the section of "Synthesis of 5-benzoyl-3-methyl-2-indolinone from 2-chloropropionyl anilide - (1)" above, under the atmosphere of an inert gas (argon gas), 2-chloropropionyl anilide (36.7 g, 0.2 mol) and anhydrous aluminum chloride (53.4 g, 0.4 mol) were heated at 130°C for 4 hours, and the temperature was gradually elevated to 150-170°C. The reaction solution was stirred with heating at the same temperature for 2 hours, and allowed to react at 180°C until the generation of hydrogen chloride gas ceased, thereby giving a reaction solution containing 3-methyl-2-indolinone.

The reaction solution was added with 1,2-dichloroethane (10 ml), and then a solution of benzoyl chloride (43.0 g, 0.3 mol) in 1,2-dichloroethane (15 ml) was added dropwise thereto over 30 minutes at around 50°C. Since the generation of heat was observed during the addition, this operation was performed while cooling on ice. After the addition, the temperature was elevated, and the reaction solution was allowed to react at about 80°C for 4 hours, then at 90°C with stirring until the generation of hydrogen chloride gas ceased. The reaction solution was cooled to room temperature, and then ethyl acetate (150 ml) wad added dropwise thereto to dissolve it.

The reaction solution was cooled on ice, and then ice cold water was added dropwise thereto slowly. During this operation, the reaction proceeded violently with generation of heat. As solids were precipitated, water was added to the reaction solution until the solids were dissolved. In this example, because crystals of 5-benzoyl-3-methyl-2-indolinone began to precipitate before the addition of water was not completed, the system was heated to dissolve the crystals and fractionated. The organic phase was washed twice with hot water (60-70°C, 100 ml), subsequently with a hot aqueous solution (100 ml) of potassium carbonate (10 g). Each aqueous phase was washed with ethyl acetate (100 ml). The organic phases were combined together and dried over magnesium sulfate (anhydrous) (10 g) and potassium carbonate (5 g). When the temperature was lowered to room temperature, crystals of 5-benzoyl-3-methyl-2-indolinone were precipitated in part. So, the reaction solution was heated and filtered. The inorganic material was washed with ethyl acetate, and the solvent was distilled away from the organic phase using an evaporator. The residue was added with methanol (30 ml), cooled on ice and allowed to stand overnight. Following filtration, the filter cake was washed with ice cold methanol (30 ml) and dried.

The yield of the crude product: 28.87 g (56.6%). Re-crystallization from methanol. The total yield of the product combined with the crystals obtained by the condensation/crystallization from the filtrate: 24.67 g (yield: 48.6%). The ¹H-NMR spectrum (solvent: CDCl₃) for the obtained sample was equivalent to that shown in Fig. 4.

### [Synthesis of 5-benzoyl-3-methyl-2-indolinone from 2-chloropropionyl anilide - (3)]

In the same manner as described above, under the atmosphere of an inert gas (argon gas), a mixture of 2-chloropropionyl anilide (36.7 g, 0.2 mol) and aluminum chloride (anhydrous) (60.5 g, 0.45 mol) was heated gradually to 125-135°C over 4 hours while stirring. The temperature was further elevated to 180°C over 1 hour. The reaction solution was stirred with heating at the same temperature until the generation of hydrogen chloride gas ceased. The reaction solution was cooled and added with 1,2-dichloroethane (20 ml), and then a solution of benzoyl chloride (43.3 g, 0.3 mol) in 1,2-dichloroethane (30 ml) was added dropwise thereto over 30 minutes. After the addition was completed, the reaction solution was stirred with heating at 75-80°C for 3 hours. Subsequently, temperature was elevated to 85-95°C, and the reaction was continued with stirring until the generation of hydrochloric acid gas ceased.

After cooling, 1,2-dichloroethane (100 ml) was added to the reaction solution, and ice blocks were further added while cooling on ice, thereby to inactivate the aluminum chloride. Even when the solids of aluminum chloride were dissolved, the reaction product was precipitated and so the separation thereof was poor. Because of this, 1,2-dichloroethane was removed from the reaction solution using an evaporator, and the residue was heated to a temperature of about 60-70°C and extracted with ethyl acetate. The extract was washed twice with hot water (60-70°C, 100 ml each) and subsequently with a hot aqueous solution (60-70°C, 100 ml) of potassium carbonate (10 g). Each aqueous phase was re-extracted with ethyl acetate (100 ml), the organic phases were combined together and then dried over magnesium sulfate (anhydride) (10 g) and potassium carbonate (5 g). The mixture was filtered while keeping the solution hot. The solvent was evaporated from the filtrate. When methanol was added to the residue, solids were precipitated and so the residue was re-crystallized from methanol. After filtration, the filter cake was washed with ice cold methanol (30 ml) and dried. Yield: 22.97 g (45.2%). The solvent was concentrated from the filtrate and cooled on ice to give additional crystals. Following filtration, the filter cake was washed with ice cold methanol (15 ml) and dried.

Yield: 2.24 g (5.4%). Total yield: 25.21 g (49.6%)/

The ¹H-NMR spectrum (solvent: CDCl₃) for the obtained sample was equivalent to that shown in Fig. 4.

### Synthesis Example for ketoprofen from 5-benzoyl-3-methyl-2-indolinone - (1)

5-Benzoyl-3-methyl-2-indolinone (2.61 g, 0.01 mol) and sodium hydroxide (0.48 g, 0.012 mol) were suspended in a mixed solvent of water (4 ml)-ethanol (1 ml), and then refluxed with heating for 8 hours under the atmosphere of nitrogen. The resulting hydrolyzed solution was added with an aqueous solution of sodium nitrite (0.84 g, 0.012 mol) in water (7 ml) at room temperature. The resulting mixture was added dropwise to a 20% aqueous solution of hydrochloric acid (20 ml) over about 1 hour while cooling on ice. After the addition, hypophosphorous acid (3.2 g, 0.048 mol) was added, and the mixture was allowed to stand for the period of day and night while stirring.

The reaction solution was extracted with ethyl acetate (20 ml), and the organic phase was washed with water. The organic phase was added with an aqueous solution of sodium hydroxide (1.0 g, 0.025 mol) in water (15 ml) to extract the acid. The aqueous phase was washed twice with ethyl acetate (10 ml), and the obtained aqueous phase was acidified with hydrochloric acid to precipitate the acid, which acid was then extracted twice with ethyl acetate (15 ml). The ethyl acetate phase was washed with water, and the ethyl acetate was evaporated from the solution to yield solids.

The solids were dissolved in ethyl acetate, and the solution was applied onto a column of silica gel (20 g), thereby removing substances appeared around the origin on the TLC. The solvent was evaporated from the solution and the residue was dried.

Yield: 2.01 g (76.6%). The Rf value of TLC and the results from the NMR analysis for the product were consistent with those for standard ketoprofen. In Fi.g 5, the ¹H-NMR chart (solvent: CDCl₃) for the produced ketoprofen is shown.

### Synthesis Example for ketoprofen from 5-benzoyl-3-methyl-2-indolinone - (2)

To 5-benzoyl-3-methyl-2-indolinone (35.4 g, 0.139 mol) was added sodium hydroxide (8.36 g, 0.209 mol) and a mixed solution of water (71 ml)-ethanol (14 ml), and the mixture was refluxed with heating for 5 hours. The resulting solution was added with sodium nitrite (11.3 g, 0.164 mol) and water (94 ml), and was added dropwise to ice cold concentrated hydrochloric acid (220 ml) while stirring vigorously over 1 hour, thereby causing diazotization. After the addition was completed, sodium hypophosphite monohydrate (63.0 g, 0.422 mol) was added to the reaction solution and continued to stir at room temperature. During this stirring, bubbling was observed and so toluene (30 ml) was added to the reaction solution to suppress the bubbling.

In the reaction, nitrogen gas was generated. When the generation of nitrogen gas ceased, the reaction was terminated. Subsequently, the reaction product was extracted with ethyl acetate (100 ml), and the ethyl acetate phase was washed with water. The ethyl acetate solution was dried over anhydrous magnesium sulfate, and filtered to remove the drying agent therefrom. Then, the solvent, ethyl acetate, was evaporated.

The residue was subjected to single distillation (boiling point: ∼ 190-230°C/1 mmHg) to give 26.26 g of a fraction (crude yield: 74.2%). This fraction was dissolved in methyl ethyl ketone (30 ml), and added with hexane (100 ml). When a loopful of pure standard ketoprofen was added to the above solution, white crystals were precipitated. Following filtration, the crystals were washed with hexane. Yeild: 22.56 g (recovery: 85.9%).

### Synthesis Example for ketoprofen from 5-benzoyl-3-methyl-2-indolinone - (3)

A mixture of 5-benzoyl-3-methyl-2-indolinone (12.7 g, 0.05 mol), sodium hydroxide (a 30% aqueous solution; 10 g, 0.075 mol) and a water (80 ml)-ethanol (20 ml) mixed solution was refluxed with heating for 5 hours to cause hydrolysis. The resulting solution was added with sodium nitrite (4.0 g, 0.058 mol), and then diluted with water to precisely give the total weight of 90 g. The solution was added dropwise to a 80% sulfuric acid (52.2 g, 0.43 mol) containing toluene (10 ml) over 45 minutes while cooling on ice, thereby causing diazotization.

Subsequently, sodium hypophosphite monohydrate (22.5 g, 0.21 mol) was added to the reaction solution, which was subjected to reduction at room temperature until the generation of nitrogen gas ceased. The reaction solution was extracted with ethyl acetate, and the extract was washed twice with water and dried over anhydrous magnesium sulfate. The resulting solution was filtered, and the solvent was evaporated therefrom.

The residue was subjected to single distillation under reduced pressure of about 1 mmHg to obtain 9.80 g of crude ketoprofen. Yield: 77.1%.

### Synthesis Example for ketoprofen from 5-benzoyl-3-methyl-2-indolinone - (4)

In each of "Synthesis Examples for ketoprofen from 5-benzoyl-3-methyl-2-indolinone (1)- (3)" above, 5-benzoyl-3-methyl-2-indolinone synthesized from isolated and purified 3-methyl-2-indolinone was used as the starting material. However, in this example, 5-benzoyl-3-methyl-2-indolinone produced in accordance with the procedures of "Synthesis of 5-benzoyl-3-methyl-2 -indolinone from 2-chloropropionyl anilide -(1)" was used as the starting material.

That is, a mixture of 5-benzoyl-3-methyl-2-indolinone (5.22 g, 0.0208 mol) sequentially produced from 2-chloropropionyl anilide, sodium hydroxide (1.04 g, 0.026 mol), water (8 ml) and ethanol (2 ml) was refluxed with heating for 8 hours. After cooling, the reaction solution was added with sodium nitrite (NaNO₂) (1.68 g) and water (14 ml). The mixture was added dropwise to an ice cold aqueous solution of hydrochloric acid (a mixture of concentrated hydrochloric acid 30 ml: water 10 ml) over 1 hour thereby causing diazotization. The resulting reaction solution was added with a sodium salt of hypophosphorous acid (NaH₂PO₂· H₂O) (9.4 g, 0.089 mol), and stirred at room temperature for 2 hours. When the generation of nitrogen ceased, the reaction was judged to be completed.

After the reaction, the reaction solution was extracted twice with ethyl acetate (20 ml). The extract was washed with water, and the acid in the solution was extracted with an aqueous solution of sodium hydroxide (2.0 g, 0.05 mol). The extract was washed with ethyl acetate, and acidified with hydrochloric acid to precipitate ketoprofen. The reaction solution was further extracted with ethyl acetate, and subjected to silica gel column chromatography (silica gel: 20 g, developing solvent: a mixed solution of equal volumes of ethyl acetate-hexane). After the compounds appeared around the origin were removed from the reaction solution, ketoprofen was dried under vacuum.

Yield: (78.7%). The ¹H-NMR spectrum of the product was the same as that of commercially available ketoprofen.

### [EFECT OF THE INVENTION]

By the specific features of the present invention, it is possible to produce ketoprofen by ordinary steps without the need for use of any extremely poisonous chemical such as prussic acid compounds or any hard-to-handle chemical such as liquid ammonium, by more shortened steps as a whole, with an excellent yield.

### [BRIEF DESCRIPTION OF DRAWINGS]

Fig. 1 shows the IR spectrum of 5-benzoyl-3-methyl-2-indolinone synthesized in Synthesis Example.
Fig. 2 shows NMR spectrum of 5-benzoyl-3-methyl-2-indolinone synthesized in Synthesis Example.
Fig. 3 shows NMR spectrum of 2-chloropripionyl anilide synthesized in Synthesis Example.
Fig. 4 shows NMR spectrum of 5-benzoyl-3-methyl-2-indolinone synthesized in Synthesis Example.

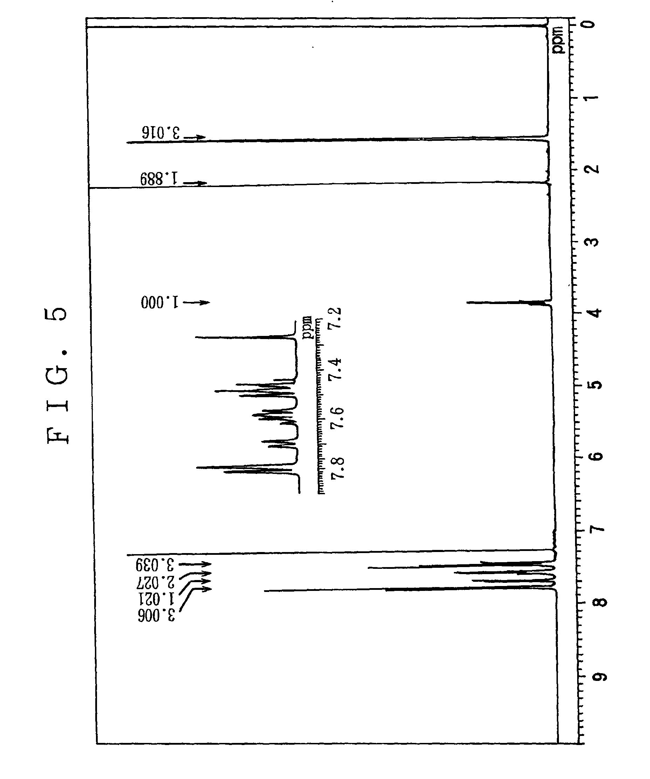

Fig. 5 shows NMR spectrum of ketoprofen synthesized in Synthesis Example.

## Claims

1. A process for producing ketoprofen, comprising the steps of:
benzoylating 3-methyl-2-indolinone with benzoyl halide to yield 5-benzoyl-3-methyl-2-indolinone;
cleaving an amido linkage that forms the 5-menbered ring moiety of 5-benzoyl-3-methyl-2-indolinone, with a basic compound; and
substituting an amino group attached to an aromatic ring of the resulting compound by a hydrogen atom to yield the ketoprofen.

2. The process for producing ketoprofen of claim 1, wherein in the step of benzoylating 3-methyl-2-indolinone with benzoyl halide to yield 5-benzoyl-3-methyl-2-indolinone, 2 moles or more of aluminum chloride and 1.5 moles or more of benzoyl chloride are used based on 1 mole of the 3-methyl-2-indolinone, and wherein the reaction temperature is 100°C or below.

3. The process for producing ketoprofen of claim 2 or 3, wherein in the steps of cleaving the amido linkage that forms the 5-menbered ring moiety of the 5-benzoyl-3-methyl-2-indolinone, with a basic compound and then substituting the amino group attached to the aromatic ring of the resulting compound by a hydrogen atom to yield the ketoprofen, an alkali metal hydroxide is used to cleave the amide linkage to cause hydrolysis of the 5-benzoyl-3-methyl-2-indolinone; a nitrite was added; the resulting reaction solution is added dropwise to an acidic solution to cause diazotization; and the diazotized product is reduced with an reducing agent.

4. The process for producing ketoprofen of claim 3, wherein the reducing agent is hypophosphorous acid or a salt compound thereof.

5. The process for producing ketoprofen of any one of claims 1 to 4, wherein 3-methyl-2-indolinone that is produced by heating 2-halopropionyl anilide represented by the formula below in the presence of aluminum halide is used without its isolation or purification, as the starting material.

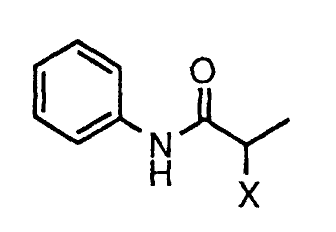

6. The process for producing ketoprofen of claim 5, wherein the aluminum halide is aluminum chloride.

7. A process for producing ketoprofen, comprising the steps of:
heating 2-halopropionyl anilide represented by the formula below in the presence of aluminum halide; and
adding benzoyl halide to the reaction system to cause benzoylation, thereby yielding the ketoprofen.
